# EUROPÄISCHE PATENTANMELDUNG

(11) **EP 0 884 301 A1**
(43) Veröffentlichungstag der Anmeldung: **16.12.1998**
(21) Anmeldenummer: 98107238.2
(22) Anmeldetag: 21.04.1998
(51) Int. Cl.: C07C 67/08, C07C 69/82

(54) **Verfahren zur Auftrennung des Rohestergemisches aus der Herstellung von Dimethylterephthalat nach dem Witten-Hercules-Verfahren**

(30) Priorität: 10.06.1997 DE 19724391
(71) Anmelder: HÜLS AKTIENGESELLSCHAFT, 45764 Marl (DE)
(72) Erfinder: Neutzler, Ulrich, 58300 Wetter (DE); Schoengen, Anton, 58452 Witten (DE)

(57) **Zusammenfassung**

Die Erfindung betrifft ein Verfahren zur Auftrennung des Rohestergemisches aus der Herstellung von Dimethylterephthalat nach dem Witten-Hercules-Verfahren durch kontinuierliche Destillation, dei sem man in mindestens eine Destillationskolonne, in der Dimethylterephthalat von Rückstand und/oder Leichtsiedern getrennt wird, Methanol einleitet und dadurch die Auftrennung mit einer Methanolyse verbindet.

## Beschreibung

Die Erfindung betrifft die Auftrennung von Rohestergemischen aus der Herstellung von Dimethylterephthalat nach dem Witten-Hercules-Verfahren, bei der die Auftrennung mit einer Methanolyse höhersiedender Bestandteile verbunden wird.

Nach dem Witten-Hercules-Verfahren wird Dimethylterephthalat (DMT) hergestellt, indem man p-Xylol (pX) in Gegenwart eines Kobalt und Mangan enthaltenden Katalysators in einem ersten Oxidationsschritt zu p-Toluylsäure (PTS) oxidiert, diese mit Methanol zum p-Toluylsauremethylester (PTE) verestert, der in einem zweiten Oxidationsschritt zum Monomethylterephthalat (MMT) oxidiert wird, das wiederum mit Methanol zu DMT verestert wird. PTE und MMT werden in derselben Veresterungsstufe gemeinsam verestert, und auch der erste und der zweite Oxidationsschritt werden ganz oder teilweise im Gemisch der beteiligten Stoffe durchgeführt. In diesem Gemisch wird auch ein Teil der PTS direkt zu Terephthalsäure (TPS) oxidiert, die zusammen mit der übrigen PTS sowie dem MMT verestert wird und ohne einen zweiten Oxidationsschritt direkt DMT ergibt. Der Oxidationsprozeß, kurz die Oxidation genannt, wird mit Sauerstoff oder Sauerstoff enthaltenden Gasen, wie Luft, diskontinuierlich oder kontinuierlich in einzelnen Reaktoren oder mehrstufigen Reaktorkaskaden durchgeführt.

Das in der Veresterungsstufe anfallende Rohestergemisch enthalt am Austritt des Veresterungsreaktors u.a.
(1) als Hauptbestandteile DMT und PTE sowie unter den Veresterungsdrücken von ca. 30 bar erhebliche Mengen an Methanol; ferner enthalten sind DMT-Isomere, die aus den Xylolisomeren m-Xylol und o-Xylol durch Oxidation und Veresterung der Dicarbonsäuren entstanden sind und sich z.T., je nach Prozeßführung, in erheblichen Mengen aufgrund der Rückführung bestimmter Ströme aus den Reinigungsstufen anreichern;
(2) nicht veresterte Säuren, wie TPS, PTS, MMT und höhermolekulare Verbindungen mit freien Carboxylgruppen:
(3) hochsiedende Ester aus aromatischen Alkoholen, wie p-Toluylalkohol (pTA), mit im Verfahren vorkommenden Säuren, wie MMT, TPS und PTS;
(4) ähnlich wie DMT siedende DMT-Vorläuferstoffe, wie die leichtersiedenden Terephthalsäurealdehydester (TAE) und p-Methoxymethylbenzoesäuremethylester (MMBME) sowie der etwas schwerer siedende p-Hydroxymethylbenzoesäuremethylester (HMBME);
(5) relativ leicht flüchtige, aber höher als DMT siedende Stoffe, wie Trimellithsäuretrimethylester (TMT), relativ leicht flüchtige Diphenyle und ähnliche Stoffe ("destillierbare Hochsieder");
(6) nicht destillierbare Anteile, wie Katalysatorbestandteile und hochkondensierte hochmolekulare Stoffe.

Die Auftrennung des Rohestergemisches ist schon wegen dessen komplexer Zusammensetzung eine anspruchsvolle Aufgabe. Bei allen technischen DMT-Verfahren wird das Rohestergemisch in der sogenannten Rohesterdestillation in einem Kolonnensystem rektifiziert und in drei Fraktionen aufgeteilt. Die leichtsiedende, den PTE enthaltende Fraktion wird in die Oxidation zurückgeführt. Die mittelsiedende wird DMT roh genannt und in weiteren Prozeßstufen, die z.B. aus Destillationen und Kristallisationen bestehen können, weiter gereinigt oder auch zur Herstellung von reiner, direkt mit Glykolen veresterbarer Terephthalsäure (TPA) verwendet. In der am höchsten siedenden Fraktion, dem "Destillationsrückstand", finden sich neben Restmengen an DMT und PTE die unter (2) bis (6) aufgeführten Stoffe. In einer Nachbehandlung werden darin enthaltene Wertstoffe gewonnen. Die Nachbehandlung ist meistens eine Methanolyse, bei der der Destillationsrückstand bei 180 bis 300°C mit Methanol behandelt wird. Dabei ist das Methanol einerseits Reaktant und andererseits Strippmedium. Bei der Methanolyse wird der Destillationsrückstand in einen mit Methanoldampf flüchtigen Anteil, der in die Oxidation zurückgeführt wird, und einen Methanolyserückstand zerlegt, aus dem noch die Katalysatorbestandteile extrahiert werden.

Die Methanolyse ist ein komplexes Geschehen bei dem u.a. die folgenden Reaktionen und Vorgänge eine Rolle spielen:
(7) Veresterung der nicht veresterten Säuren gemäß (2);
(8) Umesterung der hochsiedenden Ester aromatischer Alkohole gemäß (3) mit Methanol zu den entsprechenden Methylestern;
(9) Veretherung der gemäß (8) frei werdenden aromatischen Alkohole mit Methanol zu den entsprechenden Methylethern;
(10) Abstrippen von DMT sowie seiner ebenfalls leichtflüchtigen Vorläuferstoffe gemäß (4):
(11) Abstrippen der destillierbaren Hochsieder gemäß (5);
(12) Rückreaktionen zu den Reaktionen unter (7) und (8), die stets zu Gleichgewichten führen:
(13) Kondensationsreaktionen, die - wohl unter dem Einfluß der katalytisch wirkenden Kobaltbestandteile des Destillationsrückstands - besonders bei hohen Temperaturen ablaufen und zur Bildung hochzäher Produkte führen;
(14) thermolytische Spaltprozesse (Crackprozesse) an heißen Oberflächen und in überhitzten Anteilen des Methanolysegemisches, bei denen Kohlendioxid und vorwiegend nicht in DMT umwandelbare leichtsiedende Stoffe entstehen;
(15) Bildung schlecht löslicher. z.T. koksartiger Kobaltverbindungen aus Stoffen, die durch die Reaktionen unter (8) und (14) entstanden sind, in hochtemperierten Zonen; und
(16) Abscheidung von metallischem Kobalt durch Reduktion von Kobaltverbindungen durch Methanol bzw. darin enthaltene reduzierende Stoffe, wie Formaldehyd, namentlich an heißen Wänden.

Bei der Methanolyse kommt es darauf an, sie so zu führen, daß DMT und die ähnlich leicht flüchtigen Vorläuferstoffe gemäß (4) sowie die Methylether gemäß (9) als Wertstoffe in dem mit Methanoldampf flüchtigen Anteil gelangen, die destillierbaren Hochsieder gemäß (5), die die Oxidation stören, jedoch im Methanolyserückstand verbleiben und die unerwünschten Reaktionen unter (13) bis (16) durch möglichst niedrige Temperaturen zurückgedrängt werden.

Diese Aufgabe wird bei den Methanolyseverfahren des Standes der Technik mehr oder weniger gut, aber immer mit erheblichem apparativen und Energieaufwand gelöst. Auf die Beschreibung der verschiedenen ein- oder mehrstufigen Methanolysen kann hier verzichtet werden: in jedem Fall ist eine von der Rohesterdestillation getrennte Anlage dafür erforderlich. Es wäre daher erwünscht, wenn man die Methanolyse des Destillationsrückstandes mindestens ebenso effizient wie bisher, aber mit geringerem apparativen und Energieaufwand durchführen könnte.

Es wurde nun überraschend gefunden, daß sich das Rohestergemisch aus der Herstellung von DMT nach dem Witten-Hercules-Verfahren durch kontinuierliche Destillation vorteilhaft auftrennen läßt. wenn man in mindestens eine Destillationskolonne. in der DMT von Rückstand und/oder Leichtsiedern getrennt werden. Methanol einleitet und dadurch die Auftrennung mit einer Methanolyse verbindet.

Bei den Destillationskolonnen kann es sich um Flashdestillationskolonnen. Rektifikationskolonnen oder eine Kombination beider Typen von Destillationskolonnen handeln.

Das Methanol kann indirekt über einen mit einem Wärmetauscher versehenen Umlauf und/oder direkt, gegebenenfalls zusammen mit dampfförmigen Entspannungsprodukten des Rohestergemisches, in eine oder mehrere Destillationskolonnen eingeleitet werden Das Methanol ist einerseits Reaktant und andererseits Strippmedium. Je nach Wirkungsschwerpunkt wird zwischen dem (direkt eingeleiteten) Reaktionsmethanol und dem (indirekt eingeleiteten) Strippmethanol unterschieden. Es sollte jedoch verstanden werden, daß diese Bezeichnungen idealtypische Grenzfälle kennzeichnen. In Wirklichkeit entfaltet auch das direkt eingeleitete Reaktionsmethanol eine Strippwirkung und bewirkt das Strippmethanol in den Umläufen auch eine Methanolyse.

Bei einer vorteilhaften Variante des Verfahrens nach der Erfindung wird Methanol direkt oder indirekt in den Sumpfteil wenigstens einer der gegebenenfalls mehreren vorhandenen Destillationskolonnen eingeleitet.

Das Methanol wird vorteilhaft dampfförmig eingeleitet. Zweckmäßig verwendet man das noch dampfförmige Methanol, das bei der Entspannung des Rohestergemisches nach dem Austritt aus dem Veresterungsreaktor anfällt.

Die erfindungsgemäße elegante Kombination von Rohesterdestillation und Methanolyse bietet sich insbesondere für neu zu errichtende Anlagen an und führt dann zu einer erheblichen Reduzierung der Investitionskosten und der laufenden Betriebs- und Wartungskosten. Die maximale Temperatur in der Rektifikationszone liegt deutlich unter 250°C, so daß die unerwünschten Reaktionen gemäß (13) bis (16) weitgehend unterdrückt werden. Daher sind die Ausbeuten an DMT um einige Prozentpunkte besser als bei den besten DMT-Verfahren mit gesonderter Methanolyse, und zudem kommen Betriebsunterbrechungen infolge von Ablagerungen gemäß (15) und (16) praktisch nicht mehr vor. Die in der Oxidation störenden destillierbaren Hochsieder werden weit überwiegend im Destillationsrückstand (der zugleich Methanolyserückstand ist) zurückgehalten und erleichtern dort die Extraktion der Katalysatorbestandteile` indem sie die Viskosität des Destillationsrückstands erniedrigen.

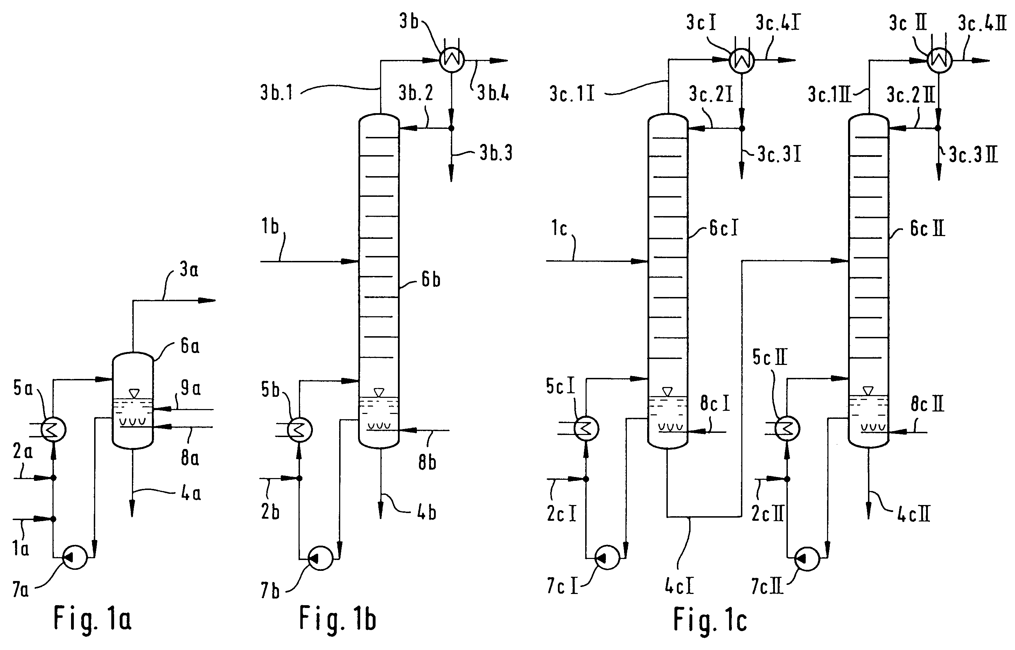

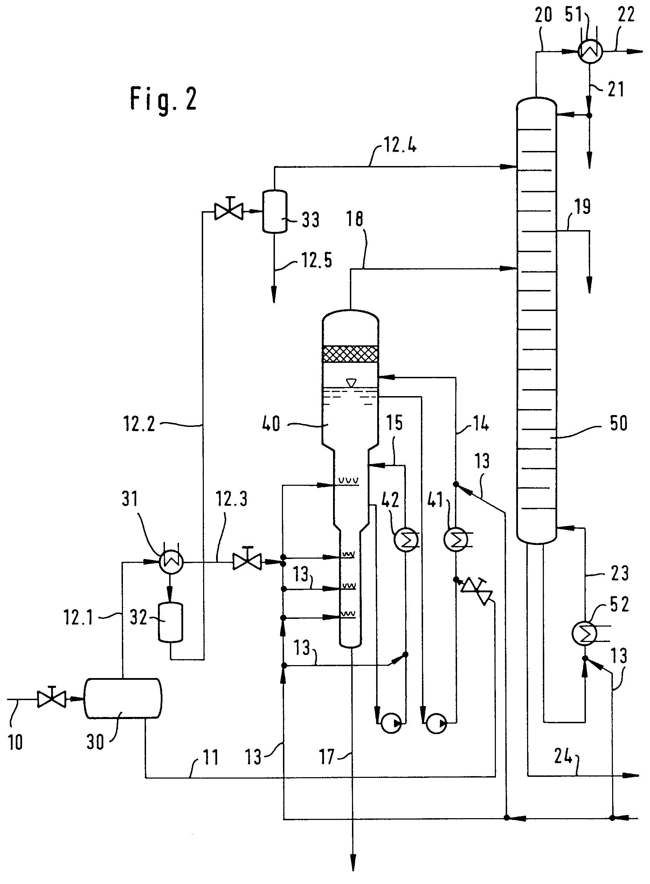

Das Verfahren nach der Erfindung kann in seiner einfachsten Form in Einrichtungen gemäß den Figuren 1a bis 1c durchgeführt werden. Der Fachmann erkennt jedoch, daß noch viele andere Ausführungsmöglichkeiten bestehen. Während die in den Figuren 1a bis 1c dargestellten Ausführungsformen auch in bestehenden Anlagen unschwer realisierbar sind, eignet sich die in Figur 2 wiedergegebene Rohesterdestillation besonders für Neuanlagen.

### Zur Figur 1a:

Bei dieser Verfahrensvariante wird das Rohestergemisch direkt (**9a**) oder über einen Umlauf indirekt (**1a**) in eine Flashdestillationskolonne **6a** eingeleitet, der gleichzeitig Methanol direkt (Reaktionsmethanol **8a**) und/oder über den Umlauf indirekt (Strippmethanol **2a**) zugeführt wird (Basis für **Anspruch 6**). Der Umlauf ist mit Wärmetauscher **5a** und Pumpe **7a** versehen. Der Brüden **3a** wird zur weiteren Aufarbeitung in andere Destillationseinrichtungen geführt. Der nicht verdampfende Rückstand **4a** wird ebenfalls in andere Destillations- und/oder Prozeßeinrichtungen gegeben. Die Pumpe **7a** kann entfallen, wenn die zur Aufrechterhaltung des Umlaufs erforderliche Volumenvergrößerung durch eine genügend große Strippmethanolmenge gewährleistet ist.

### Zur Figur 1b:

Bei dieser Verfahrensvariante wird der Rohester **1b** direkt und das Methanol direkt (Reaktionsmethanol **8b**) und/oder indirekt (Strippmethanol **2b**) in eine Rektifikationskolonne **6b** eingeleitet (Basis für **Anspruch 7**). Der Sumpfteil der Kolonne **6b** enthält einen Austritt für die nicht verdampfende Flüssigkeit **4b** und weist den erwähnten Umlauf mit Wärmetauscher **5b** und Pumpe **7b** sowie eine Reaktionsmethanoleinleitung **8b** auf.

Im Gegensatz zu Figur 1a, in der die Destillationskolonne eine Flashkolonne ist, arbeitet die Verfahrensvariante 1b mit einer Rektifikationskolonne mit einem Verstärkungs- und einen Abtriebsteil. so daß es gelingt, das Rohestergemisch **1b** in ein leichtsiedendes. z.B. den pTE enthaltendes Destillat **3b.3** und einen nicht verdampfenden Rückstand **4b** aufzutrennen. Wie in solchen Destillationen üblich, ist am Kopf ein Kondensator **3**b vorgesehen, der aus dem Brüden **3b.1** den zur Rektifikation nötigen Rücklauf **3b.2** und das Destillat **3b.3** erzeugt. Sowohl der Restbrüden **3b.4** als auch das Destillat **3b.3** können zur weiteren Aufarbeitung weiteren Prozeßstufen zugeführt werden.

Je nach Gestaltung der Destillation und der anderen Prozeßstufen kann die DMT-Fraktion sowohl im flüssigen Rückstand **4b** als auch um Destillat **3b.3** enthalten sein. Im letzteren Falle wird das Destillat **3b.3** in üblicher Weise einer weiteren Rektifikation unterworfen, wodurch die DMT-Fraktion von Leichtsiedern getrennt wird. Ist sie im flüssigen Rückstand **4b** enthalten, so kann die erfindungsgemäße Ausführung gemäß Figur 1c zur Abtrennung von Hochsiedern gewählt werden.

### Zur Figur 1c:

Bei dieser Verfahrensvariante wird das Rohestergemisch **1c** auf die erste Kolonne **6c.I** eines Systems aus mehreren Rektifikationskolonnen (**6c.I** und **6c.II**) und das Methanol wenigstens auf den Umlauf des Sumpfes der Rektifikationskolonne **6c.II** gegeben, in der der Rückstand des Rohestergemisches **1c** abgetrennt wird (**Basis für Anspruch 8**). Bei dem Verfahren gemäß Figur 1c wird das Rohestergemisch **1c** auf die erste Rektifikationskolonne **6c.I** aufgegeben, in der ein die PTE-Fraktion enthaltendes Destillat **3c.I** anfällt. Der die DMT-Fraktion enthaltende flüssige Rückstand **4cI** wird in der zweiten Rektifikationskolonne **6cII** rektifiziert, wodurch man das DMT-roh als Destillat **3c.3II** und die weitgehend methanolisierte Fraktion des Rohesters **1c** als Rückstand **4c.II** erhält. Alle anderen Teile der Figur 1c entsprechen den durch ihre Nummerierung kenntlichen analogen Teilen der Figuren 1a und 1b.

### Zur Figur 2:

Diese in ihrer Ausgestaltung vielfältig variierbare und flexible Verfahrensvariante kann durchgeführt werden, indem man das Rohestergemisch **10**, gegebenenfalls nach Teilentspannung und Abtrennung dabei frei werdender dampfförmiger Bestandteile, gemeinsam mit oder getrennt von Methanol dämpfen **13** in eine Flashkolonne **40** einführt. aus der man den nicht verdampfenden Rückstand **17** und den Kopfstrom **18** abzieht, letzteren in die Rektifikationskolonne **50** führt, in deren Sumpf indirekt über einen Umlauf Methanoldampf **13** eingeleitet wird und aus der man als Kopfstrom ein pX-reiches Destillat **21**, als Seitenstrom **19** PTE und einen DMT-reichen Rückstand **24** abzieht (**Basis für Anspruch 9**).

Das Rohestergemisch **10** kommt von der Veresterungsstufe des DMT-Verfahrens üblicherweise mit einer Temperatur von 230 bis 280°C und unter einem Druck von 25 bis 40 bar an. Es enthält im allgemeinen 40 bis 70 Gewichtsprozent DMT und dessen Isomeren, 30 bis 50 Gewichtsprozent PTE, im nicht entspannten Zustand 6 bis 8 Gewichtsprozent Methanol und wechselnde Mengen der zuvor beschriebenen anderen Bestandteile.

Das Rohestergemisch **10** kann unmittelbar in den oberen Teil der Flashkolonne **40** eingeführt werden, wird jedoch, wie in der Figur dargestellt, zweckmäßig zuerst in den Entspannungsbehälter **30** teilentspannt, üblicherweise auf 3 bis 10 bar, wobei die Temperatur auf 140 bis 230°C absinkt. Durch die Teilentspannung wird das Rohestergemisch **10 i**n einen größeren, weiterhin flüssigen Teilstrom **11** und einen kleineren, dampfförmigen Teilstrom **12** zerlegt. Auf den letzteren entfallen in der Regel, je nach Zusammensetzung des Rohestergemisches **10**, 5 bis 15 Gewichtsprozent und auf den größeren, flüssigen Teilstrom **11** dementsprechend 85 bis 95 Gewichtsprozent des Rohestergemisches **10**.

Der kleinere, dampfförmige Teilstrom **12** des Rohestergemisches **10** kann auf Kolonnendruck entspannt und unmittelbar in den unteren Teil der Flashkolonne **40** eingeführt werden. "Kolonnendruck" bedeutet hier und in der Folge den Druck, unter dem die Kolonnen **40** und **50** betrieben werden: er liegt im allgemeinen zwischen etwa 300 mbar und etwa 1.5 bar. Der größere, flüssige Teilstrom **11** wird direkt oder vorteilhaft indirekt in den oberen Teil der Flashkolonne **40** geleitet, wie später näher beschrieben wird.

Bei einer zweckmäßigen Ausführungsform der Erfindung wird der dampfförmige Teilstrom **12.1** des Rohestergemisches **10** vor seiner Entspannung auf Kolonnendruck und Einführung die Flashkolonne **40** im Wärmetauscher **31** abgekühlt, vorteilhaft auf etwa 130 bis 170°C, wodurch der größte Teil des darin enthaltenen PTE in der Vorlage **32** als Kondensat **12.2** flüssig abgeschieden wird. Der dampfförmig verbliebene Teil **12.3** des Teilstromes **12.1** wird direkt oder, wie in der Figur dargestellt, indirekt, nämlich zusammen mit Methanoldampf **13,** in den unteren Teil der Flashkolonne **40** eingeleitet. Das Kondensat **12.2** wird auf Kolonnendruck entspannt und im Trenngefäß **33** von den dabei frei werdenden Dämpfen getrennt, die überwiegend aus Methanol bestehen und als Dampfstrom **12.4** in den oberen Teil (Verstärkungsteil) der Rektifikationskolonne **50** geleitet werden. Das restliche Kondensat **12.5**, das überwiegend PTE und geringe Anteile DMT enthält, wird in die Oxidationsstufe des DMT-Verfahrens zurückgeführt.

Gleichzeitig mit der Einführung des Rohestergemisches **10** (bzw. des flüssigen Teilstromes **11** und des dampfförmigen Teilstromes **12.1** nach einer bevorzugten Ausführungsform der Erfindung) wird in den unteren Teil der Flashkolonne **40** Methanoldampf **13** eingeleitet. Diese Einleitung von Methanoldampf ist ein wesentliches Merkmal der Erfindung und hat zweierlei Wirkungen. Sie ermöglicht und fördert einerseits eine Trägerdampfdestillation (oder ein Abstrippen) von DMT, PTE und anderen, ähnlich wie DMT siedenden Bestandteilen und bewirkt andererseits eine Methanolyse, wie zuvor beschrieben.

Die Einleitung von Methanoldampf **13** in den unteren Teil der Flashkolonne **40** kann an einer oder an mehreren Stellen erfolgen. Dabei kann der Methanoldampf unmittelbar oder mittelbar, d.h. über andere Stoffströme, eingeleitet werden. So kann man, wie erwähnt, Methanoldampf zusammen mit dem dampfförmigen Teilstrom **12.1** einleiten. Eine andere, besonders vorteilhafte Einleitung ist die in mindestens einen oder aber mehrere Umlaufströme (in der Figur zwei Umlaufströme, **14** und **15**) an der Rektifikationskolonne **40**. Diese Umlaufströme werden seitlich aus der Rektifikationskolonne **40** abgezogen und oberhalb der Abzugstelle wieder eingeführt, zweckmäßig nachdem sie Wärmetauscher (**41** und **42**) durchlaufen haben, über die der Kolonne **40** die Wärmemenge zugeführt wird, die für die Trägerdampfdestillation und die Methanolyse erforderlich ist. Auf diese Weise wird in der Kolonne eine Temperatur aufrechterhalten, die je nach dem Druck und der Zusammensetzung des jeweiligen Rohestergemisches, 180 bis 260°C beträgt, wobei die Temperatur innerhalb dieses Bereiches von oben nach unten ansteigt. Die Methanolyse ist besonders intensiv, wenn man Methanoldampf in einen oder mehrere Umlaufströme einführt, weil sie dann, wie bereits erwähnt, zum Teil schon in dem Umlaufstrom bzw, den Umlaufströmen stattfindet. Infolge der Verdrängungswirkung des Methanoldampfes werden im allgemeinen zur Aufrechterhaltung des Umlaufs keine Pumpen benötigt.

Bei einer besonderen Ausführungsform des Verfahrens der Erfindung entnimmt man einen Umlaufstrom jeweils unterhalb einer indirekten (z.B. mit dem dampfförmigen Teilstrom **12.1**) oder direkten Einleitung von Methanoldampf, und führt ihn oberhalb dieser Einleitung zurück. Wenn eine solche Anordnung aus Methanoldampfeinleitung und zugehörigem Umlaufstrom mehrfach vorhanden ist, wird die Flashkolonne **40** in entsprechende "Kammern" unterteilt, in denen die Temperaturen von oben nach unten allmählich ansteigen, aber deutlich unter 250°C bleiben können.

Man verwendet für die Einleitung bzw. Einleitungen vorteilhaft zumindest u.a. Dampf von Methanol, das aus einer anderen Stufe, z.B. der Methanolverdampfung, des DMT-Verfahrens stammt. Die Temperatur des Methanoldampfes beträgt zweckmäßig 180 bis 260°C. Der Druck entspricht dem Kolonnendruck; bei Einleitung in einen Umlaufstrom muß er ausreichen, um dessen hydrostatischen Druck zu überwinden und den Verdrängungseffekt zu bewirken, der für den Umlauf erforderlich ist (sofern keine Pumpe vorhanden ist).

Die Menge des Methanoldampfes **13**, der auf den verschiedenen beschriebenen Wegen mittelbar oder unmittelbar in die Kolonne **40** gelangt, kann in weiten Grenzen variieren. Für eine wirkungsvolle Trägerdampfdestillation und eine intensive, weitgehende Methanolyse wendet man im allgemeinen die 0,01 bis 0,5-fache Gewichtsmenge Methanoldampf des zugeführten Rohestergemisches **10** an. Die Verteilung des Methanoldampfes auf die verschiedenen Einleitungsstellen ist im Prinzip beliebig. Wichtig ist lediglich, daß genügend Methanoldampf für die Trägerdampfdestillation und die Methanolyse eingesetzt wird, Bei der besonders vorteilhaften Ausführungsform, bei der Methanoldampf in einen Umlaufstrom oder mehrere Umlaufströme eingeleitet wird, kann die Einleitung von Methanoldampf auf anderem Wege (direkt oder indirekt) auf einen untergeordneten Anteil zurückgenommen werden oder ganz entfallen.

Das Rohestergemisch **10** oder bevorzugt dessen größerer, flüssiger Teilstrom **11** wird, wie bereits erwähnt, in den oberen Teil der Flashkolonne **40** entspannt. Diese Kolonne hat in ihren verschiedenen Sektionen im allgemeinen unterschiedliche Durchmesser, die der jeweiligen Querschnittsbelastung angepaßt sind. Sie hat zweckmäßig bis auf Tropfenabscheider und Methanoleinleitungen nur wenige oder gar keine Destillationsböden und -einbauten. Das Rohestergemisch **10** bzw. dessen flüssiger Teilstrom **11** wird zweckmäßig über den obersten Umlaufstrom **14** in die Flashkolonne **40** eingeführt. Er kann aber auch, wie erwähnt, direkt in diese Kolonne eingeleitet oder auf mehrere Umlaufströme verteilt werden. Die Umlaufströme (**14**, **15**) werden, wie ebenfalls bereits erwähnt, über Wärmetauscher (**41**, **42**) geleitet, die die für die Trägerdampfdestillation und die Methanolyse in der Flashkolonne **40** erforderliche Wärme liefern. Dabei sollte die Temperatur des Wärmeträgers in den Wärmetauschern nur unwesentlich, z.B. 5 bis 25°C, oberhalb der Temperatur des jeweiligen Umlaufstromes liegen, die je nach dem Kolonnendruck, im allgemeinen 180 bis 260°C beträgt. Dieses schonende Erhitzen wirkt der zuvor erwähnten Abscheidung von metallischem Kobalt entgegen. Es erfordert aber andererseits eine erhebliche Menge an Umlaufstrom, um die erforderliche Wärmemenge für die Trägerdampfdestillation und die Methanolyse einzubringen. Die Menge des Umlaufstromes **14** kann das 25 bis 100-fache der Menge des zugeführten Rohestergemisches **10** betragen.

Am Boden der Flashkolonne **40** wird der Rückstand **17** abgezogen, der bei den üblichen Temperaturen von 180 bis 260°C flüssig ist, aber bei Raumtemperatur erstarrt. Auf den Rückstand **17** entfallen üblicherweise etwa 0.5 bis 5 Gewichtsprozent des Rohestergemisches **10**. Er enthält neben den Katalysatorbestandteilen und den nicht destillierbaren hochkondensierten hochmolekularen Stoffen gemäß (6) sowie gegebenenfalls noch vorhandenen kleinen Mengen an hochzähen Stoffen gemäß (13) einen Teil der destillierbaren Hochsieder gemäß (5) und 0.5 bis 1.5 Gewichtsprozent an DMT und anderen niedermolekularen Wertstoffen, die man zwar in der Flashkolonne **40** "herausquetschen" könnte, zweckmäßig jedoch im Rückstand **17** beläßt, damit dieser in der nachfolgenden Katalysatorrückgewinnung, einer Säureextraktion bei ca. 90 bis 100°C, handhabbar bleibt. Der restliche Teil der destillierbaren Hochsieder verbleibt im Brüden **18** und damit letztlich im DMT-reichen Rückstand **24**.

Der Methanoldampf und die mit ihm ausgetragenen niedrigsiedenden Stoffe verlassen die Flashkolonne **40** über einen kurzen oberen Teil als Kopfstrom **18** mit einer Temperatur, die je nach Kolonnendruck 150 bis 250°C beträgt. Der Kopfstrom **18** wird in die Rektifizierkolonne **50** eingeleitet, die eine Glockenboden-, Siebboden- oder Füllkörperkolonne sein kann. Aus deren Verstärkungsteil werden als Seitenstrom **19** PTE und ein Teil der leichter als DMT flüchtigen Stoffe gemäß (4) abgezogen und in die Oxidationsstufe des DMT-Verfahrens zurückgeführt. Der Kopfbrüden **20** besteht überwiegend aus Methanol und Stoffen, die leichter sieden als PTE. Die Temperatur des Brüdens **20** beträgt, je nach Kolonnendruck, 25 bis 200°C. Er wird durch den Wärmetauscher **51** geleitet, in dem gegebenenfalls ein p-Xylolreiches Destillat **21** abgeschieden wird, das z.T. als Rücklauf auf die Rektifikationskolonne **50** zurückgeführt und im übrigen, gegebenenfalls nach Aufarbeitung, in die Oxidationsstufe des DMT-Verfahrens geleitet wird. Obwohl der Fachmann erkennt, daß auch andere Fahrweisen dieser Destillation möglich sind, bei denen die Rektifikationskolonne **50** mit höheren Temperaturen betrieben wird und so ein Teil der Brüdenenthalpie auf höherem Niveau zurückgewonnen werden kann, bietet die beschriebene Niedertemperaturfahrweise und die damit verbundene Xylolabtrennung wesentliche Vorteile, weil dabei die bisher übliche Xylolstrippung in den Vorstufen der Rohesterdestillation entfallen kann. Aus dem Wärmetauscher **51** tritt der Reststrom **22** aus, der überwiegend Methanol sowie geringe Mengen Wasser enthält und z.B. in die Methanolrektifikation des DMT-Verfahrens geleitet werden kann.

Die Rektifikationskolonne **50** wird durch den Wärmetauscher **52** beheizt, der in dem Umlaufstrom **23** angeordnet ist. Dessen Temperatur beträgt, je nach Kolonnendruck, im allgemeinen 150 bis 250°C. Um die Sumpftemperaturen zu senken, kann in den Umlaufstrom **23** Methanoldampf **13** von 150 bis 250°C eingeleitet werden. Die Menge des gegebenenfalls zugeführten Methanoldampfes beträgt in der Regel das 0,01 bis 0,25-fache des Gewichts des abgezogenen Rückstandes **24**, der im wesentlichen aus der DMT-roh-Fraktion besteht. Der Rest besteht überwiegend aus dem nicht im Strom **17** enthaltenen Teil der destillierbaren Hochsieder sowie aus dem ähnlich wie DMT siedenden HMBME. Der Rückstand **24** wird deshalb in nachfolgenden Destillationen und/oder Kristallisationen den Erfordernissen entsprechend weiter aufgearbeitet.

## Patentansprüche

1. Verfahren zur Auftrennung des Rohestergemisches aus der Herstellung von Dimethylterephthalat nach dem Witten-Hercules-Verfahren durch kontinuierliche Destillation, dadurch gekennzeichnet, daß man in mindestens eine Destillationskolonne, in der Dimethylterephthalat von Rückstand und/oder Leichtsiedern getrennt wird, Methanol einleitet und dadurch die Auftrennung mit einer Methanolyse verbindet.

2. Verfahren nach Anspruch 1, dadurch gekennzeichnet, daß Methanol indirekt über einen mit einem Wärmetauscher versehenen Umlauf und/oder direkt, gegebenenfalls zusammen mit dampfförmigen Entspannungsprodukten des Rohestergemisches, in eine oder mehrerer Destillationskolonnen eingeleitet wird.

3. Verfahren nach Anspruch 1 oder 2, dadurch gekennzeichnet, daß Methanol direkt oder indirekt in den Sumpfteil wenigstens einer der gegebenenfalls mehreren vorhandenen Destillationskolonnen eingeleitet wird.

4. Verfahren nach einem der Ansprüche 1 bis 3, dadurch gekennzeichnet, daß man das Methanol dampfförmig einleitet.

5. Verfahren nach einem der Ansprüche 1 bis 5, dadurch gekennzeichnet, daß das noch dampfförmige Methanol verwendet wird, das bei der Entspannung des Rohestergemisches nach dem Austritt aus dem Veresterungsreaktor anfällt.

6. Verfahren nach einem der Ansprüche 1 bis 5, dadurch gekennzeichnet, daß das Rohestergemisch direkt oder über einen Umlauf indirekt in eine Flashdestillationskolonne **6a** eingeleitet wird, der gleichzeitig Methanol direkt (Reaktionsmethanol) und/oder indirekt (Strippmethanol) zugeführt wird.

7. Verfahren nach einem der Ansprüche 1 bis 5, dadurch gekennzeichnet, daß das Rohestergemisch direkt und das Methanol direkt (Reaktionsmethanol) und/oder über einen Umlauf indirekt (Strippmethanol) in eine Rektifikationskolonne **6b** eingeleitet wird.

8. Verfahren nach einem der Ansprüche 1 bis 5, dadurch gekennzeichnet, daß das Rohestergemisch auf die erste Rektifikationskolonne **6c.I** eines Systems aus mehreren Rektifikationskolonnen gegeben und das Methanol wenigstens auf einen Umlauf des Sumpfes der Rektifikationskolonne **6c.I** gegeben wird, in der der Rückstand des Rohestergemisches abgetrennt wird.

9. Verfahren nach einem der Ansprüche 1 bis 5, dadurch gekennzeichnet, daß man das Rohestergemisch, gegebenenfalls nach Teilentspannung und Abtrennung dabei freiwerdender dampfförmiger Bestandteile, gemeinsam mit oder getrennt von Methanoldämpfen in eine Flashkolonne **40** einführt, aus der man den nicht verdampften Rückstand und den Kopfstrom abzieht, letzteren in die Rektifikationskolonne **50** führt, in deren Sumpf indirekt über einen Umlauf **23** Methanoldampf eingeleitet wird und aus der man als Kopfstrom ein p-Xylol-reiches Destillat, als Seitenstrom p-Toluylsäuremethylester und einen an Dimethylterephhalat reichen Rückstand abzieht.
